(19) Europäisches Patentamt
European Patent Office
Office européen des brevets

(11) **EP 2 170 359 B1**

(12) **FASCICULE DE BREVET EUROPEEN**

(45) Date de publication et mention de la délivrance du brevet:
**11.05.2016 Bulletin 2016/19**

(51) Int Cl.:
*A61K 36/064* (2006.01)   *A61K 31/716* (2006.01)
*A61K 31/736* (2006.01)   *A61P 3/10* (2006.01)

(21) Numéro de dépôt: **08786472.4**

(22) Date de dépôt: **25.07.2008**

(86) Numéro de dépôt international:
**PCT/EP2008/059815**

(87) Numéro de publication internationale:
**WO 2009/013357 (29.01.2009 Gazette 2009/05)**

(54) **UTILISATION D'ÉCORCES DE LEVURE POUR LE TRAITEMENT ET / OU LA PRÉVENTION DE L'HYPERINSULINÉMIE**

VERWENDUNG VON HEFEFLOCKEN ZUR BEHANDLUNG UND/ODER PRÄVENTION VON HYPERINSULINÄMIE

USE OF YEAST FLAKES FOR TREATING AND/OR PREVENTING HYPERINSULINEMIA

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **25.07.2007 FR 0756731**

(43) Date de publication de la demande:
**07.04.2010 Bulletin 2010/14**

(73) Titulaire: **Lesaffre et Compagnie**
**75001 Paris (FR)**

(72) Inventeurs:
• **LAVILLE, Martine**
**F-69392 Charly (FR)**
• **NAZARE, Julie-Anne**
**F-69007 Lyon (FR)**
• **ORIOL, Eric**
**F-94500 Champigny sur Marne (FR)**

(74) Mandataire: **Cabinet Plasseraud**
**66, rue de la Chaussée d'Antin**
**75440 Paris Cedex 09 (FR)**

(56) Documents cités:
**EP-A- 1 094 117    WO-A-2005/021015**
**FR-A- 2 825 004**

• **DATABASE WPI Week 198636 Thomson Scientific, London, GB; AN 1986-236776 XP002476554 & JP 61 167622 A (ASAHI BREWERIES LTD) 29 juillet 1986 (1986-07-29) cité dans la demande**
• **KIM YEA-WOON ET AL: "Anti-diabetic activity of beta-glucans and their enzymatically hydrolyzed oligosaccharides from Agaricus blazei" BIOTECHNOLOGY LETTERS, vol. 27, no. 7, avril 2005 (2005-04), pages 483-487, XP002476549 ISSN: 0141-5492**
• **JENKINS A L ET AL: "Depression of the glycemic index by high levels of [beta]-glucan fiber in two functional foods tested in type 2 diabetes" EUROPEAN JOURNAL OF CLINICAL NUTRITION 2002 GB, vol. 56, no. 7, 2002, pages 622-628, XP002476550 ISSN: 0954-3007**
• **DATABASE EMBASE [Online] ELSEVIER SCIENCE PUBLISHERS, AMSTERDAM, NL; novembre 2005 (2005-11), BIORKLUND M ET AL: "Changes in serum lipids and postprandial glucose and insulin concentrations after consumption of beverages with [beta]-glucans from oats or barley: A randomised dose-controlled trial" XP002476553 Database accession no. EMB-2005525568 & EUROPEAN JOURNAL OF CLINICAL NUTRITION 200511 GB, vol. 59, no. 11, novembre 2005 (2005-11), pages 1272-1281, ISSN: 0954-3007 1476-5640**

**EP 2 170 359 B1**

EP 2 170 359 B1

- **CANTERBURY & NELSON NUTRACEUTICALS : NEW ZEALAND /GRACELINC LTD.: "GLUCAGEL"[Online] - 2002 pages 1-2, XP002476551 INTERNET PUBLICATION Extrait de l'Internet: URL:http://www.nutraceuticals.org.nz/index .cf m/Member%20Profiles/Gracelinc%20Ltd> -& "GLUCAGEL TM. Barley bata-glucan and healthy blood glucose"[Online] pages 1-3, XP002476552 INTERNET PUBLICATION Extrait de l'Internet: URL:www.glucagel.com/bloodglucose.pdf>**

- **CANTERBURY & NELSON NUTRACEUTICALS : NEW ZEALAND /GRACELINC LTD.: "GLUCAGEL"[Online] - 2002 pages 1-2, XP002476551 INTERNET PUBLICATION Extrait de l'Internet:**

**Description**

La présente invention concerne l'utilisation d'écorces de levure pour le traitement et / ou la prévention de l'hyperinsulinémie.

L'insuline est une hormone hypoglycémiante sécrétée par les cellules β des îlots de Langerhans du pancréas. L'insuline permet de diminuer la glycémie, en particulier en activant le transport du glucose dans les tissus cibles (tissus musculaires, hépatiques et adipeuses).

Le diabète de type 2 se caractérise par une insulino-résistance se traduisant dans une première phase par un hyperinsulinisme. Certaines obésités sont aussi associées à un hyperinsulinisme. Dans le cas du diabète avec insulino-résistance, des médicaments qui permettent de diminuer l'excès de sucre dans le sang sans stimuler la sécrétion d'insuline peuvent être prescrits (par exemple la metformine).

Le document JP-A-61-167622 propose un agent de lutte contre le diabète à base d'une fraction cellulaire de levure de brasserie appelée paroi cellulaire dans ce document et obtenue par hydrolyse de levure de brasserie désamérisée pendant au moins 2 heures à une température de 50 à 70°C et extraction aqueuse des constituants solubles dans l'eau. Ladite fraction cellulaire de levure de brasserie présente notamment une teneur en glucanes d'environ 14,8%, une teneur en mannanes d'environ 13,9%. Ladite fraction cellulaire présente également une teneur en glycogène d'environ 24,9%. Le glycogène est un polysaccharide de réserve également présent dans les muscles et en particulier dans le foie. Ce glycogène est également une substance de réserve de la levure, utilisée par celle-ci comme source d'énergie pour assurer sa survie. Alors qu'il est un des composants principaux de la fraction cellulaire décrite dans cette demande japonaise, il ne fait pas partie de la paroi cellulaire des levures.

La demande de brevet WO2005/021015 décrit l'utilisation d'écorces de levure pour la prévention et le traitement de l'hyperglycémie et pour la stabilisation de la glycémie, lesdites écorces de levure présentant une teneur en glycogène faible et pouvant être obtenues par un procédé d'autolyse ou d'hydrolyse enzymatique simple. La teneur totale de glucanes et mannanes dans lesdites écorces de levure est d'au moins 34,0% en masse sur matières sèches.

Les β-glucanes de la paroi des cellules de levure sont essentiellement des polymères de glucose dont les unités glucose de la chaîne principale sont reliées par des liaisons β-1,3 et dont les ramifications sont reliées par des liaisons β-1,6. Les β-glucanes de levure sont insolubles et présentent une viscosité faible.

Les β-glucanes purifiés à partir de la paroi de cellules de levure possèdent des propriétés d'immunostimulation. En particulier, il a été montré que les β-glucanes de levure sont capables de se lier aux macrophages et de les activer. Les β-glucanes de levure sont étudiés pour des applications antibactériennes, antivirales et anti-tumorales (Adams D.S., Journal of Leukocyte Biology, 1997).

La présente invention a pour objet l'utilisation d'écorces de levure comme agent insulino-régulateur.

Un objet de l'invention est notamment l'utilisation d'écorces de levure dans le traitement et / ou la prévention de l'hyperinsulinémie.

La présente invention a pour objet l'utilisation d'écorces de levure pour la préparation d'une composition pharmaceutique destinée au traitement et /ou à la prévention de l'hyperinsulinémie, lesdites écorces de levure ayant :

•    une teneur totale de glucanes et mannanes d'au moins 34,0% en masse sur matières sèches, et
•    une teneur en glycogène inférieure à 10,0% en masse sur matières sèches.

Le terme « écorce de levure » se réfère à la fraction insoluble des cellules de levure, non enrichies en chrome, obtenue après une autolyse ou une hydrolyse enzymatique, essentiellement par des protéases, conduisant à la solubilisation d'au moins 50% et de préférence d'au moins 60% en masse des matières sèches des cellules de levure entières et conservant les polysaccharides de structure de la paroi cellulaire, c'est-à-dire les ß-glucanes et les mannanes, ces mannanes étant sous forme de mannoprotéines.

Cette autolyse ou hydrolyse enzymatique est conduite de manière à solubiliser l'essentiel des sucres de réserve de la cellule de levure que sont le glycogène et le tréhalose. Les écorces de levure sont obtenues par séparation de la fraction solubilisée par l'autolyse ou l'hydrolyse enzymatique, celle-ci ayant une durée de préférence d'au moins 18 heures. Les procédés préférés d'autolyse des crèmes de levure sont décrits pages 370 à 377 dans l'ouvrage de référence « Yeast Technology », 2ème édition, 1991, G. Reed et T.W. Nogodawithana, publié par Van Nostrand Reinhold, New York, ISBN 0-442-31892-8. Les écorces de levure ainsi obtenues sont ensuite typiquement séchées par un procédé classique de séchage, comme le séchage par atomisation ou le séchage sur rouleaux chauffés.

Les écorces de levure selon l'invention présentent une teneur totale en glucanes et mannanes de la paroi cellulaire (systématiquement exprimée en masse équivalente de respectivement glucose et mannose - voir méthodes de mesure ci-après) d'au moins 34,0% en masse sur matières sèches, ainsi qu'une teneur en glycogène (systématiquement exprimée en masse équivalente de glucose - voir méthodes de mesure ci-après) inférieure à 10,0% en masse sur matières sèches.

Par « hyperinsulinémie », on désigne ici à la fois une hyperinsulinémie basale et une hyperinsulinémie post-prandiale. Dans un mode de réalisation avantageux, les écorces de levure selon l'invention sont utilisées pour la prévention et/ou le traitement de l'hyperinsulinémie post-prandiale. Alternativement, les écorces de levure selon l'invention sont utilisées pour la prévention et/ou le traitement de l'hyperinsulinémie basale. Dans encore un autre mode de réalisation, les écorces de levure selon l'invention sont utilisées pour la prévention et/ou le traitement à la fois de l'hyperinsulinémie basale et de l'hyperinsulinémie post-prandiale.

L'hyperinsulinémie basale est caractérisée par une concentration plasmatique basale en insuline élevée. Le niveau basal correspond aux concentrations en insuline en dehors des prises alimentaires, notamment lorsque le sujet est à jeun. Le niveau basal physiologique est généralement inférieur ou égal à 10 mU/l chez le sujet sain mince. Ainsi, une hyperinsulinémie basale peut être caractérisée par un niveau basal, en dehors de prises alimentaires (notamment à jeun), supérieur à 10 mU/l.

L'hyperinsulinémie post-prandiale correspond à une réponse insulinique excessive suite à l'ingestion d'aliments.

L'hyperinsulinémie post-prandiale peut être mise en évidence par un test d'hyperglycémie provoquée par voie orale (HGPO). La réponse physiologique au test HGPO est caractérisée par un pic de sécrétion insulinique généralement compris de 30 et 60 mU/L chez le sujet sain mince. Ainsi, une hyperinsulinémie post-prandiale peut être caractérisée par une réponse au test HGPO avec un pic de sécrétion insulinique supérieur à 60 mU/L.

La concentration plasmatique en insuline d'un patient est mesurée par les techniques classiques bien connues de l'homme de l'art. En particulier, un test RIA (RadioImmuno-Assay) peut être réalisé sur un échantillon de plasma d'un patient (par exemple RIA, Biosource, Medgenix Diagnostics, Rungis, France).

Le traitement de l'hyperinsulinémie a pour objet de diminuer la concentration plasmatique basale en insuline et /ou de rétablir une réponse insulinique physiologique suite à l'ingestion d'aliments.

La prévention de l'hyperinsulinémie a notamment pour objet de maintenir la concentration plasmatique basale en insuline à des valeurs physiologiques et / ou de maintenir une réponse insulinique physiologique suite à l'ingestion d'aliments.

La présente invention a particulièrement pour objet le traitement et / ou la prévention de l'hyperinsulinémie post-prandiale.

La présente invention a particulièrement pour objet l'utilisation telle que définie ci-dessus, dans laquelle les écorces de levure sont du genre *Saccharomyces cerevisiae.*

Lesdites levures sont de préférence des levures de boulangerie. Une levure de boulangerie est une levure appartenant à l'espèce *Saccharomyces cerevisiae,* fabriquée à l'aide essentiellement d'une multiplication ou culture aérobie comme enseignée dans l'ouvrage de référence « Yeast Technology » cité ci-dessus et n'ayant servi avant son autolyse ou hydrolyse enzymatique à aucun usage, contrairement, par exemple, à de la levure de brasserie qui est un sous-produit de la fabrication de la bière et qui a donc servi à fabriquer de la bière avant sa récupération pour son autolyse ou hydrolyse enzymatique. Cette levure de brasserie a été multipliée essentiellement en anaérobie (la fabrication de la bière étant un processus anaérobie).

La présente invention a pour objet l'utilisation telle que définie ci-dessus, dans laquelle lesdites écorces de levure ont une teneur totale de glucanes et mannanes d'au moins 40,0% en masse sur matières sèches, de préférence au moins 45,0% en masse sur matières sèches.

De manière utile, les écorces de levure selon l'invention présentent une teneur en protéines Nx6,25 de 17,0 à 35,0% en masse sur matières sèches, de préférence de 18,0 à 26,0% en masse sur matières sèches.

L'invention a particulièrement pour objet l'utilisation telle que définie ci-dessus, dans laquelle lesdites écorces de levure ont une teneur en mannanes inférieure à 30% en masse sur matières sèches.

Dans un mode de réalisation préféré de l'invention, la teneur en mannanes dans l'écorce de levure est comprise de 20 à 26 % en masse sur matières sèches. Ce mode de réalisation correspond à des écorces de levure obtenues par autolyse ou hydrolyse enzymatique, séparation de la fraction solubilisée, et séchage de la fraction non soluble tel que décrit précédemment et dans l'exemple 1.1 (partie intitulée *Préparation des écorces de levure contenant des mannanes*). Un tel produit permet d'obtenir après un test d'hyperglycémie provoquée par voie orale (HGPO) un pic insulinique diminué par rapport à un placebo chez des patients souffrant de diabète de type 2 (voir Exemple 2).

La présente invention a notamment pour objet l'utilisation telle que définie ci-dessus, dans laquelle lesdites écorces de levure ont une teneur en mannanes inférieure à 2% en masse sur matières sèches, notamment inférieure à 1% zen masse sur matières sèches, notamment inférieure à 0,1% en masse sur matières sèches.

Dans un autre mode de réalisation préféré de l'invention, la teneur en mannanes des écorces de levure est inférieure à 0,1% en masse sur matières sèches.

Les mannanes peuvent être éliminés par un traitement alcalin à chaud. Le traitement alcalin à chaud consiste à mettre en suspension aqueuse les écorces de levure selon l'invention obtenues comme décrit ci-dessus et à chauffer la suspension en milieu alcalin entre 70°C et 100°C pendant trois heures maximum. La fraction solubilisée par ce traitement contenant une grande partie, voire la totalité des mannoprotéines est éliminée par centrifugation et lavage. La fraction non solubilisée restante est récupérée et généralement séchée.

De préférence, les mannanes sont éliminés par un traitement alcalin à chaud suivi d'un traitement acide, afin d'éliminer totalement les mannanes.

Ces traitements sont détaillés dans la partie Exemples.

De tels produits, obtenus à partir des écorces de levures précédentes soumises à un traitement alcalin à chaud et éventuellement à un traitement acide, tel que décrit précédemment (voir aussi Exemple 1.1 partie intitulée *Préparations des écorces de levures dépourvues de mannanes*), et dans lesquels les mannanes ont été éliminés en totalité ou presque (les glucanes représentant l'essentiel de la teneur totale en glucanes et mannanes), permettent d'obtenir après un test HGPO un pic insulinique diminué par rapport à un placebo, et même par rapport aux écorces de levures dans lesquelles les mannanes n'ont pas été éliminés, chez des patients souffrant de diabète de type 2 ou de surpoids (voir Exemple 2).

La présente invention a pour objet l'utilisation telle que définie ci-dessus, dans laquelle les écorces de levure ont une teneur totale en glucanes et mannanes inférieure ou égale à 90% en masse sur matières sèches, notamment inférieure ou égale à 80% en masse sur matières sèches, notamment inférieure ou égale à 70% en masse sur matières sèches.

La présente invention a plus particulièrement pour objet l'utilisation telle que définie ci-dessus, dans laquelle les écorces de levure ont une teneur totale en glucanes et mannanes de 45% à 90% en masse sur matières sèches, notamment de 52% à 80% en masse sur matières sèches, de préférence de 65% à 77% en masse, et encore de préférence de 72% à 77% en masse sur matières sèches. Ainsi, selon un mode de réalisation particulièrement avantageux, la teneur en glucanes et mannanes est comprise de 72% à 77% en masse sur matières sèches.

Notamment, dans un mode de réalisation particulier, la présente invention a pour objet l'utilisation telle que définie ci-dessus, dans laquelle la teneur en glucanes et mannanes est comprise de 72% à 77% en masse sur matières sèches et la teneur en mannanes est inférieure à 2% en masse sur matières sèches, notamment inférieure à 1% en masse sur matières sèches, notamment inférieure à 0,1 %.

Alternativement, la teneur en glucanes et mannanes peut aussi être inférieure ou égale à 55% en masse sur matières sèches, du moment qu'elle reste supérieure à 45% en masse sur matières sèches.

Ainsi, selon un mode de réalisation avantageux, la teneur en glucanes et mannanes est comprise de 52% à 57% en masse sur matières sèches.

Notamment, dans un mode de réalisation particulier, la présente invention a pour objet l'utilisation telle que définie ci-dessus, dans laquelle la teneur en glucanes et mannanes est comprise de 52% à 57% en masse sur matières sèches et la teneur en mannanes est inférieure à 2% en masse sur matières sèches, notamment inférieure à 1% en masse sur matières sèches, notamment inférieure à 0,1%.

La présente invention a pour objet l'utilisation telle que définie ci-dessus, dans laquelle les écorces de levure ont une teneur en glycogène inférieure à 8,0% en masse sur matières sèches, de préférence inférieure à 5,0% en masse sur matières sèches, de préférence inférieure à 3,0% en masse sur matières sèches, de préférence inférieure à 1,0% en masse sur matières sèches, encore de préférence inférieure à 0,1% en masse sur matières sèches.

Afin d'éliminer totalement ou quasi-totalement le glycogène de l'écorce de levure, les écorces de levure selon l'invention peuvent être soumises à un traitement alcalin à chaud, comme décrit ci-dessus, et de préférence à un traitement alcalin à chaud suivi d'un traitement acide.

Un tel traitement permet de réaliser des écorces de levure selon l'invention ayant une teneur totale en glucanes et mannanes de 45% à 90% en masse sur matières sèches, de préférence 52% à 80% en masse sur matières sèches, de préférence de 65% à 77% en masse et encore de préférence de 72 à 77% en masse sur matières sèches. Un tel traitement permet notamment de réaliser des écorces de levure qui contiennent également moins de 1,0% en masse de glycogène sur matières sèches, de préférence moins de 0,1% en masse de glycogène sur matières sèches.

La composition pharmaceutique selon l'invention peut être administrée sous différentes formes ou présentations.

La composition pharmaceutique selon l'invention comprend au moins une substance active représentée par les écorces de levures et un véhicule pharmaceutiquement acceptable.

La composition pharmaceutique selon l'invention peut comprendre une ou plusieurs autres substances actives, par exemple choisies parmi des agents hypoglycémiants ou insulino-sensibilisateurs, et notamment parmi les sulfamides hypoglycémiants, biguanide, metformine et dérivés thiazolidinedione, inhibiteurs des $\alpha$-glucosidases.

La préparation peut également comprendre une ou plusieurs vitamines, notamment choisies parmi la vitamine A, vitamine C, vitamine D, vitamine E, vitamine K, vitamines B1 (thiamine), B2 (riboflavine), B3 (niacine), B5 (acide pantothénique), B6, B8 (biotine), B9 (acide folique), B12 (cobolamine) et / ou un ou plusieurs minéraux alimentaires, notamment choisis parmi le calcium, phosphore, potassium, sodium, magnésium et fer.

La présente invention a également pour objet des compositions pharmaceutiques telles que définies ci-dessus comprenant du chrome.

La composition pharmaceutique peut comprendre un ou plusieurs excipients pharmaceutiquement acceptables.

Dans un mode de réalisation préféré, la composition pharmaceutique selon l'invention est appropriée pour une administration par voie orale.

La composition pharmaceutique peut notamment se présenter sous forme de comprimé, capsule, pilule, poudre, granulés ou suspension.

La composition pharmaceutique selon l'invention peut en particulier se présenter sous forme d'une dose d'ingestion correspondant à une quantité de matières sèches d'écorces de levure selon l'invention inférieure à 10 g, de préférence de8à9g.

Dans un mode de réalisation préféré, la présente invention a pour objet l'utilisation telle que définie ci-dessus, dans laquelle les écorces de levure présentent une teneur en matières sèches supérieure ou égale à 90%, de préférence supérieure ou égale à 94% et encore de préférence supérieure ou égale à 96 % en masse.

Une teneur en matière sèche supérieure ou égale à 90%, notamment supérieure ou égale à 94%, encore de préférence supérieure ou égale à 96% permet une meilleure conservation des écorces de levure, notamment une meilleure stabilité bactériologique et une meilleure stabilité vis-à-vis de réactions indésirables d'origine enzymatique ou non.

La présente invention a pour objet l'utilisation d'écorces de levure telle que définie ci-dessus pour la prévention et / ou le traitement de l'hyperinsulinémie, qu'il s'agisse d'hyperinsulinémie basale ou post-prandiale, quelle que soit l'origine de l'hyperinsulinémie.

La présente invention a particulièrement pour objet l'utilisation d'écorces de levure telle que définie ci-dessus pour la prévention et / ou le traitement de l'hyperinsulinémie associée à une insulino-résistance, à un surpoids, à l'obésité, ou au syndrome métabolique, chez tout type de patient souffrant d'hyperinsulinémie de cette origine.

Cependant, bien que l'invention soit destinée à la prévention et au traitement de tout type d'hyperinsulinémie, chez tout type de patient, dans un mode de réalisation avantageux, les écorces selon l'invention sont utilisées pour la préparation d'un médicament destiné à la prévention et / ou au traitement de l'hyperinsulinémie chez un patient non diabétique. Dans ce cas, la présente invention a particulièrement pour objet l'utilisation d'écorces de levures telle que définie ci-dessus pour la prévention et / ou le traitement de l'hyperinsulinémie associée à une insulino-résistance, à un surpoids, à l'obésité, ou au syndrome métabolique, toujours chez des sujets non-diabétiques.

Alternativement, les écorces selon l'invention peuvent également être utilisées pour la préparation d'un médicament destiné à la prévention et / ou au traitement de l'hyperinsulinémie chez un patient diabétique de type 2. La présente invention a alors particulièrement pour objet l'utilisation d'écorces de levures telle que définie ci-dessus pour la prévention et / ou le traitement de l'hyperinsulinémie associée à une insuline-résistance, à un surpoids, à l'obésité, ou au syndrome métabolique, chez un sujet diabétique de type 2.

Par « diabète de type 2 », on désigne également le diabète non insulino-dépendant (DNID). Le diabète de type 2 est caractérisé par une glycémie à jeun supérieure à 1,26 g/L.

Lorsque la glycémie à jeun est entre 1,10 g/L et 1,26 g/L, on parle d'anomalie de la glycémie.

La présente invention a notamment pour objet l'utilisation d'écorces de levure telle que définie ci-dessus pour la prévention et / ou le traitement de l'hyperinsulinémie dans les premiers stades du diabète de type 2.

Par « insulino-résistance », on désigne le défaut de réponse des tissus insulino-dépendants à l'action de l'insuline. Le pancréas continue alors de sécréter de l'insuline dont la concentration plasmatique devient trop élevée.

Par « surpoids » et « obésité », on désigne un excès de poids. Chez l'adulte, le surpoids est caractérisé par un Indice de Masse Corporelle (IMC) compris de 25 à 30 kg/m$^2$ et l'obésité par un Indice de Masse Corporelle (IMC) supérieur à 30 kg/m$^2$. L'Indice de Masse Corporelle d'un individu est défini par la formule suivante :

$$IMC = \frac{masse(kg)}{(taille(m))^2}$$

L'expression « surpoids ou obésité chez des sujets non-diabétiques » désigne un surpoids ou une obésité tels que définis ci-dessus chez des sujets dont la glycémie est correctement régulée.

En particulier, la concentration plasmatique en glucose de sujets non diabétiques à jeun est inférieure à 1,26 g/L (7 mmol/L), notamment inférieure 1,10 g/L à (6,1 mmol/L).

L'expression «syndrome métabolique» est utilisée pour désigner un ensemble de facteurs de risques de développement de troubles cardiovasculaires, accidents vasculaires cérébraux (AVC) et diabètes de type 2.

Le diagnostic du syndrome métabolique repose sur l'évaluation de plusieurs paramètres, dont notamment le tour de taille, la concentration en cholestérol et en triglycérides, la glycémie à jeun, l'insulinémie et la tension artérielle.

En particulier, selon le NCEP-ATPIII (Nation Cholestérol Education Program

- Adult treatment Panel III), le syndrome métabolique est diagnostiqué si trois ou plus des facteurs à risque suivants sont présents :
- tour de taille supérieur à 88 cm chez la femme et supérieure à 102 cm chez l'homme,
- concentration en cholestérol HDL inférieure à 1 mmol/l chez l'homme et 1,2 mmol/l chez la femme,
- concentration en triglycérides supérieure ou égale à 1,7 mmol/l,

- glycémie à jeun supérieure ou égale à 6,1 mmol/l,
- tension artérielle supérieure à 130 mm Hg / 85 mm Hg.

L'hyperinsulinémie post-prandiale peut notamment jouer un rôle dans le développement du syndrome métabolique.

La présente invention concerne également une méthode pour le traitement et/ou la prévention de l'hyperinsulinémie chez un patient, comprenant l'administration au patient d'une composition pharmaceutique selon l'invention.

La méthode pour le traitement et/ou la prévention de l'hyperinsulinémie peut en particulier être une méthode pour le traitement et/ou la prévention de l'hyperinsulinémie associée à une insulino-résistance, à un surpoids, à l'obésité, ou au syndrome métabolique. Dans un mode de réalisation avantageux, le patient est un patient non diabétique. Alternativement, le patient peut également être un patient diabétique de type 2.

L'invention concerne en particulier une telle méthode dans laquelle la composition pharmaceutique est administrée au patient par voie orale.

Les différentes méthodes selon l'invention peuvent en particulier comprendre l'administration au patient de la composition pharmaceutique en une dose journalière correspondant à 1 à 10 g, de préférence de 8 à 9 g d'écorces de levure selon l'invention, ladite dose journalière pouvant être administrée en une seule dose ou à un seul moment d'administration, comme par exemple au petit-déjeuner, ou encore en plusieurs doses partielles, c'est-à-dire de manière étalée dans la journée.

Les méthodes selon l'invention peuvent également comprendre au moins une étape de vérification de la concentration basale en insuline du patient et / ou de sa réponse insulinique suite à l'ingestion d'aliments ou à un test HGPO, après l'administration de la composition pharmaceutique en aigu ou en chronique.

Les méthodes selon l'invention peuvent en plus comprendre une étape de mesure de la concentration basale en insuline du patient et / ou d'évaluation de sa réponse insulinique suite à l'ingestion d'aliments ou à un test HGPO, avant l'administration de la composition pharmaceutique ci-dessus en aigu ou en chronique.

La présente invention concerne également une méthode pour diminuer ou stabiliser la concentration plasmatique basale en insuline chez un patient souffrant d'hyperinsulinémie basale, comprenant l'administration audit patient d'une quantité efficace d'une composition pharmaceutique selon l'invention.

La méthode pour diminuer ou stabiliser la concentration plasmatique basale en insuline peut en particulier viser un patient souffrant d'hyperinsulinémie basale associée à une insulino-résistance, à un surpoids, à l'obésité, ou au syndrome métabolique. Dans un mode de réalisation avantageux, le patient est un patient non diabétique. Alternativement, le patient peut également être un patient diabétique de type 2.

La présente invention concerne également une méthode pour diminuer le pic de sécrétion insulinique suite à l'ingestion d'aliments chez un patient souffrant d'hyperinsulinémie post-prandiale, comprenant l'administration audit patient d'une quantité efficace d'une composition pharmaceutique selon l'invention.

La méthode pour diminuer le pic de sécrétion insulinique suite à l'ingestion d'aliments peut en particulier viser un patient présentant une hyperinsulinémie post-prandiale associée à une insulino-résistance, à un surpoids, à l'obésité, ou au syndrome métabolique. Dans un mode de réalisation avantageux, le patient est un patient non diabétique. Alternativement, le patient peut également être un patient diabétique de diabète de type 2:

DESCRIPTION DES FIGURES

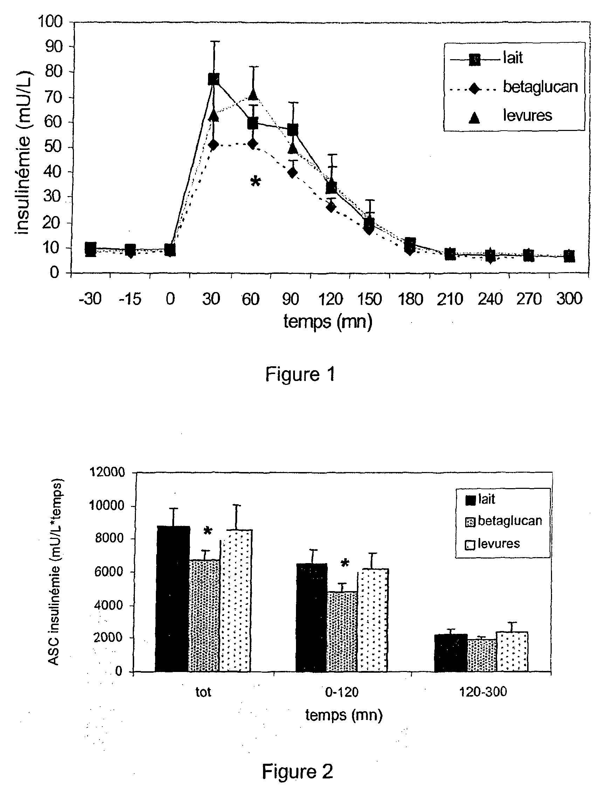

Figure 1 : Cinétique d'évolution de l'insulinémie post-prandiale chez des sujets en surpoids (n=12). Les résultats de 3 journées d'exploration sont donnés en moyenne +/- ESM. L'insulinémie des sujets témoins ayant ingéré du lait figure en ligne pleine et carrés, des sujets ayant ingéré des écorces de levure dépourvues en mannanes (bétaglucanes) en tirets et losanges, et des sujets ayant ingéré les écorces de levure avec mannanes en pointillés et triangles. La concentration en insuline (en mU/l) est donnée en fonction du temps en minutes (min).

Figure 2 : Evolution de l'insulinémie post-prandiale représentée en aire sous courbe (ASC)) chez des sujets en surpoids (n=12). Les résultats de 3 journées d'exploration sont donnés en moyenne +/- ESM. L'aire sous courbe obtenue chez les sujets témoins ayant ingéré du lait figure en noir, chez les sujets ayant ingéré des écorces de levure dépourvues en mannanes (bétaglucanes) en pointillés serrés et chez les sujets ayant ingéré les écorces de levure avec mannanes en pointillés laches. L'aire d'insulinémie (en mU/l*temps) est donnée pour la période 0-300 minutes (tot), la période 0-120 minutes et la période 120-300 minutes.

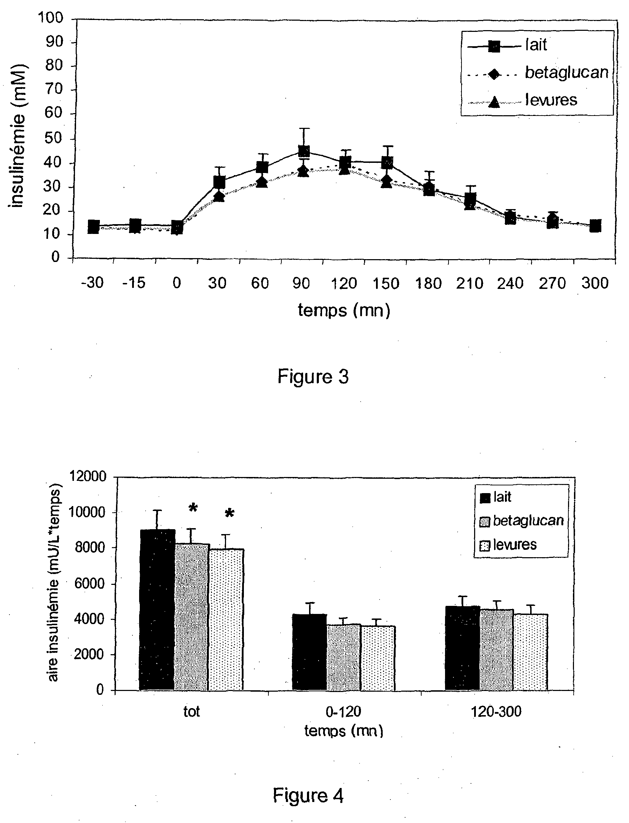

Figure 3 : Cinétique d'évolution de l'insulinémie post-prandiale chez des sujets diabétiques de type 2 (n=11).

Les résultats de 3 journées d'exploration sont donnés en moyenne +/- ESM. L'insulinémie des sujets témoins ayant ingéré du lait figure en ligne pleine et carrés, des sujets ayant ingéré des écorces de levure dépourvues en mannanes (bétaglucanes) en tirets et losanges, et des sujets ayant ingéré les écorces de levure avec mannanes en pointillés et triangles. La concentration en insuline (en mU/l) est donnée en fonction du temps en minutes (min).

Figure 4 : Evolution de l'insulinémie post-prandiale représentée en aire sous courbe (ASC)) chez des sujets diabétiques de type 2 (n=11).

Les résultats de 3 journées d'exploration sont donnés en moyenne +/- ESM. L'aire d'insulinémie des sujets témoins ayant ingéré du lait figure en noir, des sujets ayant ingéré des écorces de levure dépourvues en mannanes (bétaglucanes) en pointillés serrés et des sujets ayant ingéré les écorces de levure avec mannanes en pointillés laches. L'aire d'insulinémie (en mU/l*temps) est donnée pour la période 0-300 minutes (tot), la période 0-120 minutes et la période 120-300 minutes.

EXEMPLES

Exemple 1 : Obtention d'écorces de levure selon l'invention

1. Matériel et Méthodes

*Préparation des écorces de levure contenant des mannanes*

Une crème aqueuse (c'est-à-dire une suspension de cellules de levure dans l'eau) de *Saccharomyces cerevisiae,* ayant une teneur en matières sèches entre 12 et 18% en masse est soumise à une hydrolyse à l'aide des enzymes endogènes desdites cellules de levure, éventuellement avec ajout de protéases exogènes aux cellules de levure, telle que par exemple la papaïne. L'hydrolyse est effectuée à 50°C pendant 24 heures, de manière à solubiliser au moins 60% en masse des matières sèches des cellules de levure.

De manière pratique, en général, les autolyses ou hydrolyses enzymatiques selon l'invention sont réalisées entre 45°C et 55°C pendant 18 à 36 heures, sans l'emploi d'aucune enzyme pouvant solubiliser les glucanes ou les mannoprotéines.

La fraction solubilisée est séparée de la fraction insoluble par plusieurs étapes successives de centrifugation et de lavage avec de l'eau.

La fraction insoluble est séchée sur des tambours chauffés jusqu'à une teneur en matières sèches de 95% en masse. Les agglomérats formés sont enlevés par tamisage et on obtient des écorces de levure selon l'invention.

*Préparations des écorces de levures dépourvues de mannanes*

Les écorces de levure obtenues précédemment sont mises en suspension aqueuse et chauffées en milieu alcalin entre 70°C et 100°C pendant trois heures maximum. En particulier, la suspension en milieu aqueux alcalin sodé est chauffée à 85°C pendant deux heures.

La fraction solubilisée par ce traitement est éliminée, la fraction non-solubilisée restante étant récupérée, lavée et généralement séchée. La fraction solubilisée contenant les mannoprotéines et également le glycogène, est éliminée par centrifugation et lavage.

Les écorces de levures obtenues après le traitement alcalin à chaud sont de préférence soumise à un traitement acide afin d'éliminer toute trace de mannoprotéines. La fraction insoluble est ainsi acidifiée avec de l'acide phosphorique ou acétique, puis mise en incubation à 80°C pendant une à deux heures. La fraction solubilisée contenant les résidus de mannoprotéines, glycogène et également quelques lipides est éliminée par centrifugation et lavage.

*Mesure de la teneur en glycogène*

On ajoute à un échantillon de 20 mg d'écorces de levure sèches, c'est-à-dire à une teneur en matières sèches d'au moins 90% en masse, 0,5ml de $Na_2CO_3$ 0,25M et on maintient ce mélange à 95°C pendant 4 heures.

Le mélange est ensuite porté à un pH de 5,2 par addition de 0,3ml d'acide acétique 1M et de 1,2ml d'acétate de sodium 0,2M et en mélangeant les ingrédients. On ajoute de l'eau distillée jusqu'à un volume total de 2ml.

0,5 ml de la suspension ainsi obtenue sont incubés pendant 15 heures en présence d'un excès d'amyloglucosidase d'*Aspergillus niger,* telle que commercialisée par la société ROCHE sous le numéro Cat. No. 102 857, à 55°C.

Après centrifugation, on dose le glucose libéré par dosage enzymatique.

Le dosage enzymatique de glucose est décrit notamment dans le manuel « Methods of Biochemical Analysis and Food Analysis - using Single Reagents », publié par BOEHRINGER MANNHEIM GmbH Biochemica, © 1989, pages 50 à 55, et est de préférence réalisé en utilisant le « Test-Combination D-Glucose/- Fructose », Cat. No. 139 106 de la filiale de la société ROCHE : BOEHRINGER MANNHEIM GmbH / R-BIOPHARM GmbH à Darmstadt, Allemagne.

La quantité (en mg) de glucose ainsi dosée correspond à la quantité de glycogène présente dans l'échantillon exprimée en masse équivalente de glucose.

*Mesure de la teneur totale en glucanes et mannanes*

On soumet un échantillon de 20 mg d'écorces de levure sèches, c'est-à-dire à une teneur en matières sèches d'au moins 90% en masse, à une hydrolyse acide en le mélangeant avec 20 ml de HCl 2N, et on maintient le mélange en tube à vis fermé pendant 4 heures à 103°C en étuve avec agitation toutes les 15mn.

Ensuite, on neutralise la solution acide ainsi obtenue et on dose par voie enzymatique la quantité de glucose et respectivement de mannose dans la solution neutralisée.

Ce dosage enzymatique de glucose et mannose est également décrit aux pages 50 à 55 du manuel cité ci-dessus et est de préférence réalisé en utilisant le « Test-Combination » Cat. No. 139 106.

On fait la différence entre d'une part la quantité de glucose (exprimée en mg) dosée selon cette méthode et la quantité de glucose (également exprimée en mg) dosée pour ces écorces de levure par la méthode pour la mesure de la teneur en glycogène ci-dessus.

Cette différence (en mg) entre les deux quantités de glucose dosées correspond à la quantité totale de glucanes présente dans l'échantillon, cette quantité étant exprimée en masse équivalente de glucose.

La quantité (en mg) de mannose dosée correspond à la quantité totale de mannanes présente dans l'échantillon, cette quantité étant exprimée en masse équivalente de mannose.

2. Résultats

Les écorces de levure ainsi obtenues présentent une teneur en matières sèches de 95% en masse.

La composition des écorces de levure contenant des mannanes et des écorces de levure dépourvues de mannanes (obtenues par traitement alcalin à chaud) est donnée dans le tableau 1.

Tableau 1 : Composition des écorces de levures selon l'invention

|  | Ecorces de levures contenant des mannanes | | Ecorces de levure dépourvues de mannanes | |
|---|---|---|---|---|
|  | Analyse en g pour 100 g de produit | Apport énergétique en Kcal/g | Analyse en g pour 100 g de produit | Apport énergétique en Kcal/g |
| Glucides dont<br>G lucanes<br>Mannanes | 49,9<br><br>28,2<br>21,7 | 2,046 | 55,1<br><br>55,1<br>0 | 2,259 |
| Protéines | 23,6 | 1,298 | 2,2 | 0,121 |
| Lipides | 11,8 | 1,109 | 21,6 | 2,03 |

Pour les deux types d'écorces de levure, la teneur en glycogène était inférieure à 10% en masse sur matières sèches.

Exemple 2 : Effet des écorces de levure selon l'invention sur la concentration en insuline de sujets en surpoids non diabétiques et de sujets diabétiques non insulino-dépendants (DNID)

Par la suite, les écorces de levures contenant des mannanes sont qualifiées d' «écorces de levure » et les écorces de levures dépourvues de mannanes sont qualifiées de « bétaglucanes ».

1. Matériel et Méthodes

*Sujets, caractéristiques et taille de l'échantillon*

24 sujets volontaires de 2 types ont été recrutés: 12 sujets en surpoids sains et 12 sujets diabétiques de type 2 (hommes ou femmes).

Pour les sujets en surpoids, les critères d'inclusion sont : un index de masse corporelle compris entre 25 et 30

kg/m$^2$ (bornes incluses), âge compris entre 30 et 65 ans (bornes incluses), glycémie à jeun normale < 7 mmol/l, HbAlc < 6%, cholestérol total ≤7.0 mmol/l, triglycérides ≤4.0 mmol/l.

Pour les sujets diabétiques de type 2, les critères d'inclusion sont : âge compris entre 30 et 65 ans (bornes incluses), traitement par metformine et/ou sulfamides hypoglycémiants et/ou glinides et/ou glitazones depuis au moins 3 mois, HbA1c < 10%, cholestérol total ≤7.0 mmol/l, triglycérides ≤4.0 mmol/l.

Les principaux critères d'exclusion sont : diabète de type 1, diabète de type 2 traité par insuline ou acarbose, insulinopénie, insuffisance rénale ou hépatique chronique, antécédents de maladie gastro-intestinale chronique, pathologie endocrinienne, traitement qui peut interférer avec le métabolisme des glucides et le comportement alimentaire, intolérance au lait de vache, claustrophobie (mesures calorimétriques sous Canopy®), femmes enceintes, sujets grands consommateurs de produits contenus dans la liste des aliments à proscrire.

Seul un sujet diabétique s'est révélé insulinopénique lors de la première hyperglycémie provoquée et a donc été exclu de l'étude car ne répondant pas aux critères d'inclusion définis. Les 23 autres sujets ont complété l'étude.

*Tests d'hyperglycémies provoquées par voie orale (HGPO)*

L'essai s'est déroulé au cours de 3 journées d'hospitalisation séparées par un intervalle de 7 à 14 jours. Chaque sujet a bénéficié de chacune des 3 HGPO dans un ordre aléatoire tiré au sort.

8g d'écorces de levure et de 9g de bétaglucanes sont utilisés dans les tests HGPO. Il est à noter que la consommation d'écorces de levures à la dose prévue de 8 g ne dépasse pas les niveaux de consommation observés pour les levures de panification et les levures-aliments.

Les produits sont dilués dans 167 ml d'une solution de $^{13}$Cglucose à 30% contenant 50g de $^{13}$Cglucose. Pour le repas-test placebo, les 167 ml de solution de $^{13}$Cglucose seront additionnés de 28ml de lait entier et 50 ml de lait demi-écrémé afin d'obtenir un apport énergétique équivalent aux 8 g d'écorces de levure et aux 9 g de bétaglucanes.

La journée métabolique se divise en deux parties : une période basale (T-120 à T0) au cours de laquelle les conditions basales (à jeun) sont mesurées, puis la période post-prandiale de 4h45 (T15 - T300) consécutive à l'HGPO.

Au cours du test HGPO, le patient ingère 50 g de $^{13}$Cglucose à T0. En fonction du tirage au sort, il est éventuellement ajouté extemporanément à la solution soit les écorces de levure, soit les bétaglucanes, soit le lait. La solution doit être ingérée en moins de 10 minutes. Le patient doit rester en décubitus pendant les 5 heures suivantes, en évitant de dormir. Au cours de la journée d'exploration, le sujet peut consommer de l'eau (300ml) maximum.

Les concentrations plasmatiques en insuline sont déterminées en basal (entre T-120 et T0) et dans la période post-prandiale de T15 à T300. Les prélèvements veineux sont immédiatement centrifugés et le plasma récupéré pour le dosage de l'insuline. Le dosage de l'insuline plasmatique est réalisé par radioimmuno-essai (Medgenix Diagnostics®, Rungis, France).

*Traitement statistique*

Les résultats sont présentés sous forme moyenne par groupe +/- écart standard à la moyenne (ESM). Les résultats obtenus avec les 3 conditions (écorces de levure, bétaglucanes de levures et placebo (lait)) sont comparés deux à deux à l'aide du test non paramétrique de Wilcoxon sur séries appariées. Pour effectuer ces tests statistiques, le logiciel Statview (Statview, Abacus Concepts, Berkeley, CA) est utilisé.

2. Résultats

On note une bonne tolérance digestive des deux produits de levure testés par les sujets.

*Evolution de l'insulinémie au cours des 3 HGPO chez les sujets surpoids*

L'augmentation de l'insulinémie en réponse à la charge orale en glucose est significativement moins élevée en présence de bétaglucanes qu'avec le placebo.

Le pic insulinique est significativement plus faible en présence de bétaglucanes qu'avec le placebo (63.0 +/- 7.7 mU/l versus 91.6 +/- 13.4 mU/l pour la condition lait (p<0.01)) (figure 1). L'aire sous la courbe de l'insulinémie en présence de bétaglucanes est plus faible par rapport au placebo de manière significative (p< 0.05) (figure 2 et tableau 2).

S'agissant des écorces de levure, une tendance à une diminution du pic insulinémique et de l'aire sous courbe est observée par rapport au placebo.

Tableau 2 : Pic insulinique en mU/l et aires sous la courbe d'insulinémie en mU/l*temps chez les sujets en surpoids. Les résultats sont exprimés en moyenne +/-ESM (n=12). p<x = différence significative par rapport au placebo (lait).

| Insulinémie surpoids | Lait | Ecorces de levure | Bétaglucanes |
|---|---|---|---|
| Pic insulinique | 91.6 +/- 13.4 | 84.2 +/- 13.2 | 63.0 +/- 7.7 p < 0.01 |
| Aire Sous Courbe 0-300 min | 8715 +/- 1130 | 8570 +/- 1444 | 6736 +/- 591 p< 0.05 |
| Aire Sous Courbe 0-120 min | 6499 +/- 903 | 6214+/-960 | 4816 +/- 504 p<0.05 |
| Aire Sous Courbe 120-300 min | 2216 +/- 335 | 2356 +/- 548 | 1920 +/- 158 |

*Evolution de l'insulinémie au cours des 3 HGPO chez les sujets diabétiques de type 2*

Chez les sujets diabétiques de type 2, une diminution de la réponse insulinique est également observée en présence de bétaglucanes, ainsi qu'en présence des écorces de levure.

Le pic insulinique est réduit de manière significative à la fois en présence de bétaglucanes et d'écorces de levure (respectivement 44.2 +/- 6.0 mU/l et 43.8 +/- 4.7 mU/l versus 56.1 +/- 8.8 mU/l pour le lait, p<0.05) (figure 3 et tableau 3).

L'aire sous courbe est également réduite de manière significative en présence d'écorces de levure et de bétaglucanes par rapport au placebo (figure 4 et tableau 3).

Tableau 3 : Pic insulinique en mU/l et aires sous la courbe d'insulinémie en mU/l*temps chez les sujets diabétiques de type 2. Les résultats sont exprimés en moyenne +/- ESM (n=11). p<x = différence significative par rapport au placebo (lait).

| Insulinémie diabétiques | Lait | Ecorces de levure | Bétaglucanes |
|---|---|---|---|
| Pic insulinique | 56.1 +/- 8.8 | 43.8 +/- 4.7 p< 0.05 | 44.2 +/- 6.0 p<0.05 |
| Aire Sous Courbe 0-300 min | 9049 +/- 1070 | 7947 +/- 821 p= 0.05 | 8248 +/- 878 p< 0.05 |
| Aire Sous Courbe 0-120 min | 4325 +/- 619 | 3644 +/- 381 | 3708 +/- 404 |
| Aire Sous Courbe 120-300 min | 4725 +/- 587 | 4303 +/- 502 | 4540 +/- 498 |

Les résultats d'insulinémie des deux populations, les sujets en surpoids non diabétiques et les sujets diabétiques de type 2, montrent donc une diminution significative de la réponse insulinique lors de l'ingestion d'écorces de levure dépourvues de mannanes. Il en est de même lors de l'ingestion d'écorces de levure contenant des mannanes chez les sujets diabétiques de type 2.

**Revendications**

1.  Ecorces de levure pour leur utilisation dans le traitement et/ou la prévention de l'hyperinsulinémie chez un patient non-diabétique, où l'hyperinsulinémie est associée à une insulino-résistance, à un surpoids, à l'obésité ou au syndrome métabolique, lesdites écorces de levure étant obtenues par autolyse ou hydrolyse enzymatique de cellules de levure, et ayant :

    - une teneur totale de glucanes et mannanes d'au moins 34,0% en masse sur matières sèches, et
    - une teneur en glycogène inférieure à 10,0% en masse sur matières sèches.

2.  Ecorces de levure pour l'utilisation selon la revendication 1, dans laquelle ladite autolyse ou hydrolyse enzymatique de cellules de levure consiste à:

    - soumettre une suspension aqueuse de cellules de levure à une hydrolyse en utilisant les enzymes endogènes desdites cellules de levure et éventuellement en ajoutant des protéases exogènes auxdites cellules de levure,
    - séparer la fraction insoluble produite, et
    - sécher la fraction insoluble séparée pour obtenir des écorces de levure.

3. Ecorces de levure pour l'utilisation selon la revendication 1 ou revendication 2, dans laquelle ladite autolyse ou hydrolyse enzymatique est effectuée à une température comprise entre 45°C et 55°C pendant 18 à 36 heures.

4. Ecorces de levure pour l'utilisation selon la revendication 2 ou de la revendication 3, dans laquelle ladite autolyse ou hydrolyse enzymatique de cellules de levure consiste en outre à:

   - chauffer une suspension aqueuse d'écorces de levure obtenues dans un milieu alcalin à une température comprise entre 70°C et 100°C pendant 3 heures au plus ; et
   - séparer la fraction insoluble pour obtenir des écorces de levure dépourvues de mannanes.

5. Ecorces de levure pour l'utilisation selon l'une quelconque des revendications 1 à 4, dans laquelle les écorces de levure sont du genre *Saccharomyces cerevisiae.*

6. Ecorces de levure pour l'utilisation selon l'une quelconque des revendications 1 à 5, dans laquelle lesdites écorces de levure ont une teneur totale de glucanes et mannanes d'au moins 40,0% en masse sur matières sèches.

7. Ecorces de levure pour l'utilisation selon l'une quelconque des revendications 1 à 6, dans laquelle lesdites écorces de levure ont une teneur en mannanes inférieure à 30% en masse sur matière sèches.

8. Ecorces de levure pour l'utilisation selon l'une quelconque des revendications 1 à 7, dans laquelle lesdites écorces de levure ont une teneur en mannanes inférieures à 2% en masse sur matières sèches.

9. Ecorces de levure pour l'utilisation selon l'une quelconque des revendications 1 à 8, dans laquelle les écorces de levure ont une teneur totale en glucanes et mannanes inférieure ou égale à 90% en masse sur matières sèches.

10. Ecorces de levure pour l'utilisation selon la revendication 12, dans laquelle la teneur en glucanes et mannanes est inférieure ou égale à 80% en masse sur matières sèches.

11. Ecorces de levure pour l'utilisation selon l'une quelconque des revendications 1 à 10, dans laquelle les écorces de levure ont une teneur totale en glucanes et mannanes de 45% à 90% en masse sur matières sèches.

12. Ecorces de levure pour l'utilisation selon la revendication 11, dans laquelle la teneur totale en glucanes et mannanes est de 52% à 80% en masse sur matières sèches.

13. Ecorces de levure pour l'utilisation selon l'une quelconque des revendications 1 à 12, dans laquelle les écorces de levure ont une teneur en glycogène inférieure à 8,0% en masse sur matières sèches.

14. Ecorces de levure pour l'utilisation selon la revendication 13, dans laquelle la teneur en glycogène est inférieure à 5,0% en masse sur matières sèches.

15. Ecorces de levure pour l'utilisation selon quelconque l'une des revendications 1 à 14, dans laquelle les écorces de levure présentent une teneur en matières sèches supérieure ou égale à 90%.

**Patentansprüche**

1. Heferinden zu ihrer Verwendung bei der Behandlung und/oder Verhinderung von Hyperinsulinämie eines Nicht-Diabetes-Patienten, wobei die Hyperinsulinämie verbunden ist mit einer Insulinresistenz, Übergewicht, Fettsucht oder metabolischem Syndrom, wobei die Heferinden erhalten werden durch enzymatische Autolyse oder Hydrolyse von Hefezellen, und aufweisen:

   - einen Gesamtgehalt an Glucanen und Mannanen von mindestens 34,0 Masse-%, bezogen auf die Trockenmasse, und
   einen Glykogengehalt unter 10,0 Masse-%, bezogen auf die Trockenmasse.

2. Heferinden zur Verwendung nach Anspruch 1, worin die enzymatische Autolyse oder Hydrolyse von Hefezellen umfasst:

- Unterziehen einer wässrigen Suspension von Hefezellen einer Hydrolyse unter Verwendung von endogenen Enzymen der Hefezellen und gegebenenfalls Hinzugeben exogener Proteasen zu den Hefezellen,
- Abtrennen der erzeugten unlöslichen Fraktion, und
- Trocknen der abgetrennten unlöslichen Fraktion, um Heferinden zu erhalten.

3. Heferinden zur Verwendung nach Anspruch 1 oder Anspruch 2, worin die enzymatische Autolyse oder Hydrolyse bei einer Temperatur, umfassend zwischen 45 °C und 55 °C für 18 bis 36 Stunden durchgeführt wird.

4. Heferinden zur Verwendung nach Anspruch 2 oder Anspruch 3, worin die enzymatische Autolyse oder Hydrolyse von Hefezellen weiterhin umfasst:

- Erwärmen einer wässrigen Suspension von Heferinden, erhalten in einem alkalischen Milieu bei einer Temperatur, umfassend zwischen 70 °C und 100 °C, für höchstens 3 Stunden; und
- Abtrennen der unlöslichen Fraktion, um Heferinden zu erhalten, die frei von Mannanen sind.

5. Heferinden zur Verwendung nach einem der Ansprüche 1 bis 4, worin die Heferinden von der Gattung *Saccharomyes cerevisiae* sind.

6. Heferinden zur Verwendung nach einem der Ansprüche 1 bis 5, worin die Heferinden einen Gesamtgehalt an Glucanen und Mannanen von mindestens 40,0 Masse-%, bezogen auf die Trockenmasse, aufweisen.

7. Heferinden zur Verwendung nach einem der Ansprüche 1 bis 6, worin die Heferinden einen Gesamtgehalt an Mannanen unter 30 Masse-%, bezogen auf die Trockenmasse, aufweisen.

8. Heferinden zur Verwendung nach einem der Ansprüche 1 bis 7, worin die Heferinden einen Gesamtgehalt an Mannanen unter 2 Masse-%, bezogen auf die Trockenmasse, aufweisen.

9. Heferinden zur Verwendung nach einem der Ansprüche 1 bis 8, worin die Heferinden einen Gesamtgehalt an Glucanen und Mannanen unter oder gleich 90 Masse-%, bezogen auf die Trockenmasse, aufweisen.

10. Heferinden zur Verwendung nach Anspruch 12, worin der Gehalt an Glucanen und Mannanen unter oder gleich 80 Masse-%, bezogen auf die Trockenmasse, ist.

11. Heferinden zur Verwendung nach einem der Ansprüche 1 bis 10, worin die Heferinden einen Gesamtgehalt an Glucanen und Mannanen von 45 Masse-% bis 90 Masse-%, bezogen auf die Trockenmasse, aufweisen.

12. Heferinden zur Verwendung nach Anspruch 11, worin der Gehalt an Glucanen und Mannanen von 52 Masse-% bis 80 Masse-%, bezogen auf die Trockenmasse, ist.

13. Heferinden zur Verwendung nach einem der Ansprüche 1 bis 12, worin die Heferinden einen Gesamtgehalt an Glycogen unter 8,0 Masse-%, bezogen auf die Trockenmasse, aufweisen.

14. Heferinden zur Verwendung nach Anspruch 13, worin der Gesamtgehalt an Glycogen unter 5,0 Masse-%, bezogen auf die Trockenmasse, ist.

15. Heferinden zur Verwendung nach einem der Ansprüche 1 bis 14, worin die Heferinden eine Trockenmasse über der gleich 90 % aufweisen.

**Claims**

1. Yeast cell walls for use thereof in the treatment and/or prevention of hyperinsulinaemia in a nondiabetic patient, wherein the hyperinsulinaemia is associated with insulin resistance, excess weight, obesity or metabolic syndrome, said yeast cell walls being obtained by autolysis or enzymatic hydrolysis of yeast cells and having:

- a total glucan and mannan content of at least 34.0% by mass on dry matter, and
- a glycogen content of less than 10.0% by mass on dry matter.

2. Yeast cell walls for the use according to Claim 1, wherein said autolysis or enzymatic hydrolysis of yeast cells consists in:

- subjecting an aqueous suspension of yeast cells to hydrolysis using enzymes endogenous to said yeast cells and optionally adding proteases exogenous to said yeast cells,
- separating the insoluble fraction produced, and
- drying the separated insoluble fraction to obtain yeast cell walls.

3. Yeast cell walls for the use according to Claim 1 or Claim 2, wherein said autolysis or enzymatic hydrolysis is carried out at a temperature of between 45°C and 55°C for 18 to 36 hours.

4. Yeast cell walls for the use according to Claim 2 or Claim 3, wherein said autolysis or enzymatic hydrolysis of yeast cells further consists in:

- heating an aqueous suspension of yeast cell walls obtained in an alkaline medium at a temperature of between 70°C and 100°C for at most 3 hours; and
- separating off the insoluble fraction to obtain yeast cell walls devoid of mannans.

5. Yeast cell walls for the use according to any one of Claims 1 to 4, wherein the yeast cell walls are of the *Saccharomyces cerevisiae* genus.

6. Yeast cell walls for the use according to any one of Claims 1 to 5, wherein said yeast cells walls have a total glucan and mannan content of at least 40.0% by mass on dry matter.

7. Yeast cell walls for the use according to any one of Claims 1 to 6, wherein said yeast cell walls have a mannan content of less than 30% by mass on dry matter.

8. Yeast cell walls for the use according to any one of Claims 1 to 7, wherein said yeast cell walls have a mannan content of less than 2% by mass on dry matter.

9. Yeast cell walls for the use according to any one of Claims 1 to 8, wherein the yeast cell walls have a total glucan and mannan content of less than or equal to 90% by mass on dry matter.

10. Yeast cell walls for the use according to Claim 12, wherein the glucan and mannan content is less than or equal to 80% by mass on dry matter.

11. Yeast cell walls for the use according to any one of Claims 1 to 10, wherein the yeast cell walls have a total glucan and mannan content from 45% to 90% by mass on dry matter.

12. Yeast cell walls for the use according to Claim 11, wherein the total glucan and mannan content is from 52% to 80% by mass on dry matter.

13. Yeast cell walls for the use according to any one of Claims 1 to 12, wherein the yeast cell walls have a glycogen content of less than 8.0% by mass on dry matter.

14. Yeast cell walls for the use according to Claim 13, wherein the glycogen content is less than 5.0% by mass on dry matter.

15. Yeast cell walls for the use according to any one of Claims 1 to 14, wherein the yeast cell walls have a dry matter content of greater than or equal to 90%.

Figure 1

Figure 2

Figure 3

Figure 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- JP 61167622 A
- WO 2005021015 A

### Littérature non-brevet citée dans la description

- **ADAMS D.S.** *Journal of Leukocyte Biology,* 1997
- **G. REED ; T.W. NOGODAWITHANA.** Yeast Technology. Van Nostrand Reinhold, 1991
- Methods of Biochemical Analysis and Food Analysis - using Single Reagents. BOEHRINGER MANNHEIM GmbH Biochemica, 1989, 50-55